# EUROPEAN PATENT SPECIFICATION

(11) **EP 2 595 617 B1**
(45) Date of publication and mention of the grant of the patent: **07.12.2016**
(21) Application number: 11761177.2
(22) Date of filing: 18.07.2011
(51) Int. Cl.: A61K 31/165, A61K 31/357, A61K 31/4168, A61M 35/00, A61P 25/02, A61K 9/00, A61K 47/32, A61K 9/08

(54) **TOPICAL TREATMENT OF NEUROPATHIC PAIN AND METHODS OF DIAGNOSIS**
TOPISCHE BEHANDLUNG VON NEUROPATHISCHEM SCHMERZ UND DIAGNOSEVERFAHREN
TRAITEMENT TOPIQUE DE LA DOULEUR NEUROPATHIQUE ET MÉTHODES DE DIAGNOSTIQUE

(30) Priority: 19.07.2010 US 365656 P
(43) Date of publication of application: 29.05.2013
(73) Proprietor: Arcion Therapeutics, Inc., Baltimore, MD 21224 (US)
(72) Inventor: CAMPBELL, James, N., Baltimore, Maryland 21230 (US)
(74) Representative: Hoffmann Eitle
(86) International application number: PCT/US2011/044358
(87) International publication number: WO 2012/012333

(56) References cited:
- WO-A2-99/39693
- WO-A2-2007/056460
- US-A- 5 447 947
- US-A- 6 147 102
- US-A1- 2004 101 582
- PETERSEN KARIN L ET AL: "Assessment of cutaneous nociceptor function by capsaicin response test during and after acute herpes zoster.", NEUROLOGY, vol. 60, no. 5 Supplement 1, 11 March 2003 (2003-03-11), page A155, XP009153252, & 55TH ANNUAL MEETING OF THE AMERICAN ACADEMY OF NEUROLOGY; HONOLULU, HAWAII, USA; MARCH 29-APRIL 05, 2003 ISSN: 0028-3878

## Description

### RELATED APPLICATIONS

This application claims priority under 35U.S.C. 119 to U.S.S.N. 61/365,656 *"Topical Treatment of Neuropathic Pain and Methods of Diagnosis* " filed July 19, 2010 by James N. Campbell.

### FIELD OF THE INVENTION

The invention is directed to compounds for use in the treatment of pain associated with diseases of the nervous system, including length dependent and other neuropathies, and painful diabetic neuropathy such as may result from diabetes and other conditions.

### BACKGROUND OF THE INVENTION

A variety of diseases can affect the peripheral nervous system. Many of these disorders are not painful, but if the pain signaling system is affected, then pain may result. One of the prototype painful neuropathies stems from diabetes. One of the most common effects on the nervous system is a length dependent neuropathy. This means that the longer the sensory axon the more likely the axon may be affected. Given that the axons that go to the feet are the longest primary afferents in the body, these fibers are affected first. As the disease progresses, other axons shorter in length are affected. The length dependent neuropathies may be caused by a variety of diseases. The most common (60-70%) is diabetes. These neuropathies may also be caused by a large variety of disorders and include kidney disease, hormonal imbalances, vitamin deficiencies, alcoholism, autoimmune disorders, toxins, chemotherapy, and infections (e.g., AIDS). These are not sympathetically maintained pain.

Other neuropathies are not length dependent and may be associated with etiologies such as herpes zoster infection (shingles and post-herpetic neuropathy), nerve trauma, and nerve compression. Complex regional pain syndrome (CRPS) is a poorly understood disorder that has many of the features of neuropathic pain. This disorder may be associated with a frank lesion of the nervous system (Type II) or not (Type I). Though a specific nerve lesion may not be obvious in Type I CRPS, it is strongly suspected that an underlying neuropathic disorder underlies the genesis of this problem. Oral drugs such as amitriptyline, duloxetine, gabapentin and pregabalin are recommended as first-line treatment options for treatment of neuropathic pain on the basis of the results of randomized clinical trials.

As knowledge about neuropathy and pain increases it is evident that different pathophysiological mechanisms are at play. Some are peculiar to the specific form of neuropathic pain, but others are shared across the various neuropathic pain disorders. Thus, in the case of different treatments, such as with oral gabapentin, some patients respond well and many others do not across the broad spectrum of neuropathic disorders. Heretofore, it has not been clear why one patient responds and the other does not. The clinician is forced to undertake an empiric trial of the drug to determine whether the drug will work. This is clearly suboptimal and leads to suffering and delay in finding efficacious treatment.

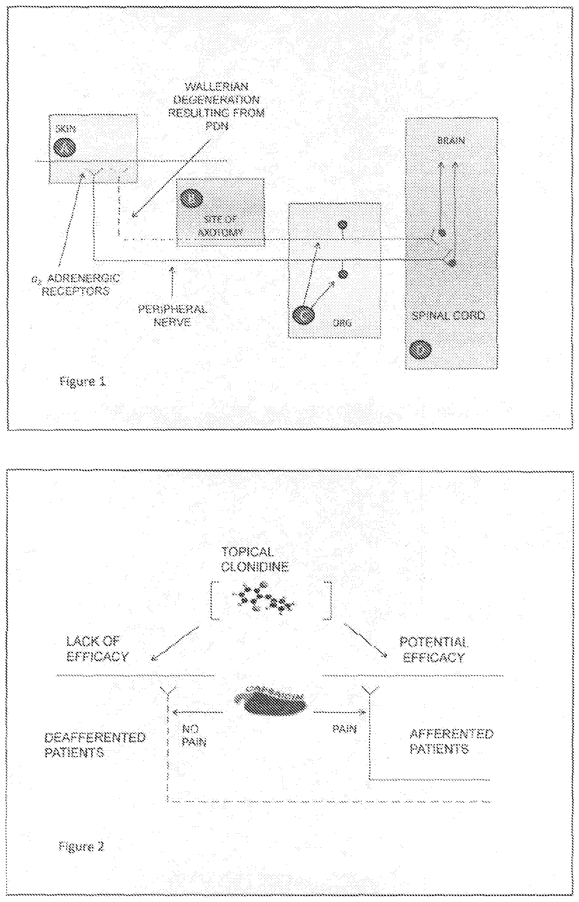

As a specific example, painful diabetic neuropathy (PDN) is more accurately considered to be a collection of diseases. This is logical given the protean manifestations of diabetic neuropathy and for that matter the other 'opathies of diabetes as well (retinopathy, nephropathy, vasculopathy, etc). A simple conception of PDN is illustrated in Figure 1. The letters A through D, refer to sites of putative pain generation (the origin of the abnormal pain signaling). In many patients the pain likely arises within the central nervous system (e.g., the dorsal horn of the spinal cord). In others the signaling likely arises from the dorsal root ganglion (site C), or some other point along the peripheral nerve fiber proximal to the skin. What has been unclear is the extent to which pain signaling may be present at site A. What has also been unclear is the role of nociceptors in the skin.

Pain often develops from diseases that affect the somatosensory system. One disease that is often implicated is diabetes mellitus. Diabetes may affect the nervous system in different ways but one of the classical disorders is a length dependent neuropathy. Here the longer sensory nerve fibers are preferentially involved in a neuropathy which is associated with both degeneration and a sensitization of nociceptors. The classic feature is burning pain typically involving the feet since the axons to the feet represent the longest primary afferents in the body. This problem may occur early or late in the disease, as well as in so-called pre-diabetes which is a condition representing a disorder of glucose metabolism without strictly meeting the criteria for diabetes mellitus. It is appreciated that diabetes is but one cause of a length dependent neuropathy. For example, it is clear that chemotherapy used to treat cancer can also induce a length dependent neuropathy. The painful symptoms that accompany these disorders, including an idiopathic small fiber neuropathy, are nearly identical with that seen in diabetes mellitus. Treatments directed at the diabetes mellitus itself may help slow the progression of the neuropathy but do not necessarily address the pain. There are no known treatments for idiopathic length dependent small fiber neuropathy. Certain chemotherapeutic drugs induce a length dependent neuropathy associated with pain. This pain may limit dosing and thus affect the adequacy of the cancer treatment.

Clearly there is a great need to have therapies that address the pain symptoms. Systemic treatments of pain include use of opioids, anticonvulsants, antidepressants, and membrane stabilizers. These therapies suffer from two drawbacks: they may relieve the pain inadequately and they may be poorly tolerated due to side effects. Systemic therapies can be given orally or by patches applied to the skin.

Some prior attempts have been made to treat painful diabetic neuropathy with clonidine, a potent alpha₂-adrenergic partial agonist used primarily for the treatment of hypertension. Clonidine has been applied topically to areas remote to the painful area as an alternative to oral delivery for effecting systemic delivery. For example, in a placebo-controlled crossover pain trial in patients with painful diabetic neuropathy, no statistically significant difference between patients receiving systemic clonidine administered with transdermal patches and patients receiving placebo patches was observed (Zeigler et al. Pain 48:403-408 (1992)). In a follow-up placebo controlled pain study in similar patients with painful diabetic neuropathy, transdermal patches delivering systemic levels of clonidine were evaluated using a two-stage enriched enrollment design (Byas-Smith et al. Pain 60: 267-274 (1995)). Twelve of forty-one patients (29%) who completed the initial course of treatment were considered clonidine responders. These twelve clonidine responders were then rechallenged in a second placebo controlled study which used the highest dosage available with the transdermal patch system. The pain reduction relative to placebo tended to be modest although statistically significant (p<0.015). The site of action of clonidine was not determined in this study. In principal the site of action could be central or peripheral. In other pain conditions a central analgesic action of clonidine has been determined. It is important to emphasize that this treatment involved systemic delivery of clonidine with a transdermal patch applied remotely to the painful area which is expected to result in systemic blood levels exceeding 0.2 ng/ml. Other therapies with oral medications have been shown to be effective to treat neuropathic pain. These include gabapentin, pregabalin, and duloxetine. Each of these therapies work only in certain patients. Moreover, systemic side effects may make these therapies of limited value. Dosing was limited because of the systemic delivery of the clonidine.
WO 2007/056460 A discloses compositions, and methods of use thereof, are provided for the treatment of painful neuropathy by local or topical delivery of compounds that interact with a-adrenergic receptors, especially an alpha2 adrenergic agonist such as clonidine, to the entire painful area such that the need for systemic dosing is minimized. The compounds are delivered to or adjacent to painful areas in patients with painful length dependent neuropathy, and other neuropathies that affect the pain signaling fibers in the skin. A preferred compound for the treatment of patients with length dependent neuropathy is clonidine applied in a transdermal patch, gel, ointment, lotion, liposomal formulation, cream, or emulsion, wherein the concentration is sufficient to provide an effective dose in the painful area or immediately adjacent areas.
US 5 447 947 A describes that sympathetically maintained pain is treated topically by administering to the site where sympathetically maintained pain is present an alpha -adrenergic antagonist, alpha -1-adrenergic antagonist, alpha 2 adrenergic agonist, or other drug that depletes or blocks synthesis of sympathetic norepinephrine. Examples demonstrate relief of pain by application of phentolamine or clonidine.
US 6 147 102 A discloses that sympathetically maintained peripheral neuropathic pain syndromes are relieved by topically applying, to the affected region of a patient suffering from such pain, a pain relieving amount of an aqueous gel comprising clonidine, a water-gelling amount of a pharmaceutically acceptable gelling agent and having a physiologically tolerable pH value. It is further stated that pain relief was achieved with applied amounts of clonidine in the range of about 2 milligrams per day to about 6 mg per day.
US 2004/101582 A describes methods and compositions for the topical or transdermal treatment of neuropathy and more particularly, transdermal or topical compositions including a combination of ingredients that are said to provide a surprising degree of effective relief from the symptoms of peripheral neuropathy and methods for administering the compositions to treat various neuropathies.

Other than an empiric trial of simply looking to see if a given patient responds to the treatment, no technique has been provided to identify the responsive patients. Moreover, none of the existing therapies has any means evolved to determine who will respond to what treatment. This is frustrating because it may take months of trial and error to determine the best treatment for a given patient.

A further issue is that there is still only a rudimentary understanding about how and why neuropathic pain occurs. For example, with diabetes of similar severity some patients develop neuropathy and others do not. In some cases the neuropathy is dominated by motor findings and in others sensory systems are affected primarily. As well, some patients have small fiber sensory neuropathy while others have large fiber neuropathy (tactile sense, loss of vibratory sense, and proprioception). Finally pain may be the dominant symptom of the neuropathy where in others there is no pain at all. Therefore it is clear that neuropathy is not a "monolithic" disease state but instead refers to a collection of diseases. The ability to distinguish these different diseases is logically linked to different responses to therapy.

Heretofore, the treatment of neuropathic pain, including PDN, is largely empiric. The clinician tries the drug and if it works the treatment is continued.

It is therefore an object of the present invention to provide compositions to effectively treat or alleviate pain in length dependent or other neuropathies, as may be associated with diabetes, by topical local delivery to the painful area of an alpha-2 adrenergic agonist, and to provide a means of diagnosis and selection of patients who are responders to such treatment.

### SUMMARY OF THE INVENTION

Alpha-2 adrenergic agonists such as clonidine may be used to treat the pain associated with painful diabetic neuropathy (PDN) and other neuropathies only in a subset of these patients. In one group nociceptors are expressed functionally in the skin and are likely sensitized. This group responds to topical clonidine with significant relief because the targeted alpha-2 adrenergic receptor is expressed in the skin in the nociceptors, activity in which generates the patient's pain. Many patients with PDN have severe degeneration and the targeted nociceptors are not expressed in the skin. The presence of the targeted nociceptors may be determined by topical application of a TRPV1 agonist such as capsaicin which induces a sensation of burning pain. Patients who detect the capsaicin as a painful stimulus applied in the area at or near the painful area have expression in the skin of the requisite targeted nociceptors and the targeted alpha-2 adrenergic receptors. The test is referred to as a capsaicin challenge test. Responders can be treated for pain due to length dependent or other neuropathy by local or topical delivery of concentrations of compounds that are agonists of the alpha- 2 adrenergic receptors, especially an alpha₂ adrenergic agonist such as clonidine, to the painful area, without producing systemic levels as appropriate for treating disorders such as hypertension. Based on these findings, the present invention, as defined in the appended claims, has been accomplished.

### BRIEF DESCRIPTION OF THE DRAWINGS

Figure 1 is a schematic showing that the neural signaling of pain may arise at different locations along the neuroaxis. For illustrative purposes, two peripheral nerve fibers are shown. One innervates the skin (shown on the left), while the second has degenerated to the site shown in B. The abnormal signaling leading to pain may arise at the level of the skin (A), the point of the "axotomy" induced by peripheral nerve disease (B), the level of the dorsal root ganglion (DRG, site C), or the spinal cord dorsal horn (D). Topical clonidine targets α₂ receptors located on terminals of nociceptors in the skin. Efficacy of this therapy is predicated on the presence of functional and likely sensitized nociceptors in the skin.
Figure 2 is a schematic illustrating the model of how the response of patients to topical capsaicin is expected to predict the response to topical clonidine. In diseases such as painful diabetic neuropathy, some patients have severe small fiber neuropathy such that nociceptor innervation of the skin is missing (left). In others the skin has ample innervation (right). If the skin lacks "pain" fiber innervation, then clonidine has no target, as the clonidine effects are mediated via activation of α₂ receptors located on the nociceptors. The topical capsaicin stimulus will evoke pain only in the instance where nociceptors are expressed on the skin. Therefore assessment of pain to topical capsaicin can be used to predict responses to topical clonidine as a treatment of neuropathic pain. The dashed line represents a "pain" fiber that has undergone Wallerian degeneration. The solid line on the right illustrates an intact nociceptive (pain) fiber.

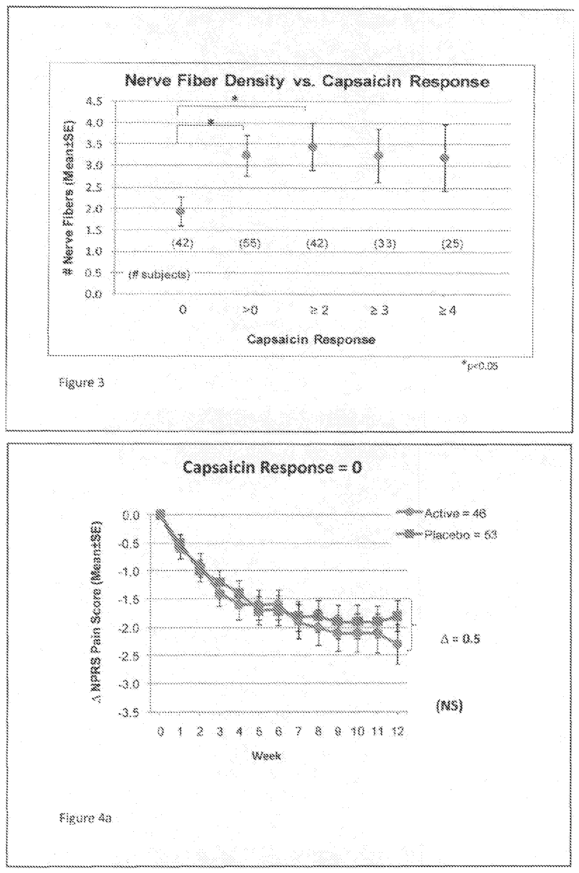

Figure 3 is a graph of intraepidermal nerve counts in the area of neuropathy as a function of ratings to a topical capsaicin applied to a nearby area in subjects with painful diabetic neuropathy. The 0.1% capsaicin stimulus was applied to the pretibial area for 30 minutes at which time the evoked pain level was recorded using a 0-10 numerical pain rating scale. The skin biopsy was analyzed using a standard marker of epidermal nerve fibers (PGP 9.5). In patients who did not feel pain in response to the capsaicin stimulus, the nerve fiber count was significantly lower compared to patients who detected pain to capsaicin with a score greater than zero (number of subjects shown in parentheses).

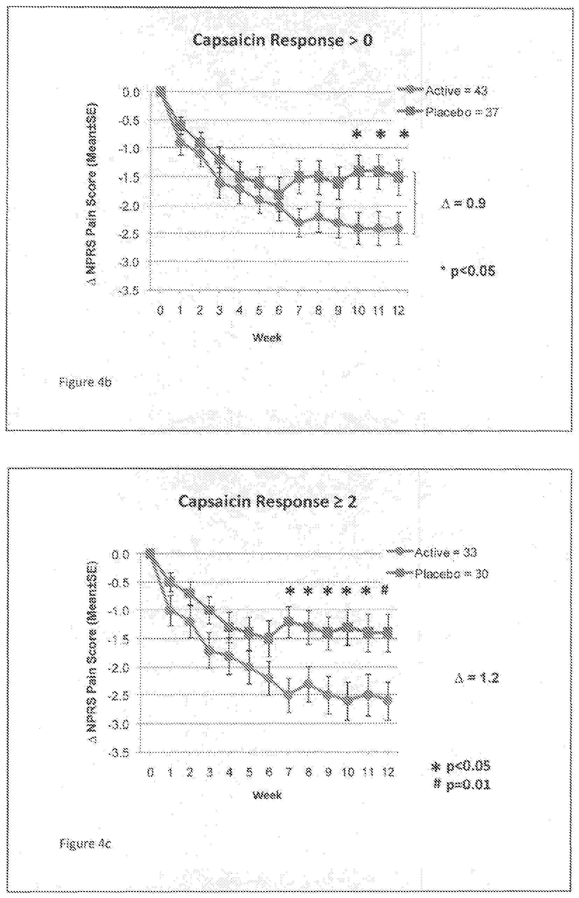

Figures 4A-4C are graphs of the data from 179 subjects with painful diabetic neuropathy with pain in the feet enrolled in a 12 week double blinded randomized clinical trial to assess the analgesic efficacy of topical clonidine applied to the painful area. Pain was assessed each day using a pain diary using a standardized numerical pain rating scale (NPRS). The average pain for each week was calculated and the difference from baseline was determined (week x minus baseline). Figures 4A, B, and C show the change in pain over time for placebo and active (clonidine treatment group) for different subgroups based on ratings of the 30 minute capsaicin challenge test. Shown in Figure 4A are the results in subjects who felt no pain to the capsaicin stimulus. Figures 4B and4C show the results for subjects with capsaicin responses greater than 0 and ≥2, respectively. The baseline pain scores were imputed for missing data arising from premature withdrawal from the study (BOCF, baseline observation carried forward). The numbers of subjects for each group are shown in the upper right-hand corner. These data demonstrated that clonidine had no efficacy over placebo in patients who did not detect the capsaicin stimulus. However, in subjects who detected capsaicin (pain rating>0), or even more clearly, had a capsaicin pain rating of two or more (0 to 10 scale), clonidine was significantly superior to placebo in relieving the diabetic pain.

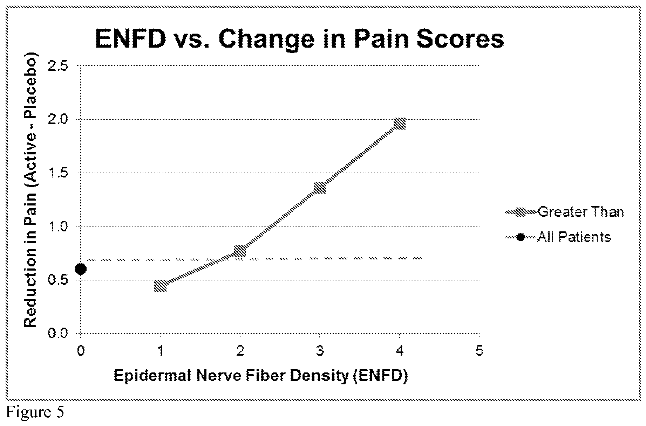

Figure 5 indicates the reduction in pain (week 12 of treatment compared to baseline) in the Clonidine Gel group versus the Placebo group as a function of nerve fiber density (nociceptors) in the superficial layer of the skin (epidermis). The data were collected in a 97 patient subgroup as part of a larger double blind randomized study in 180 patients with painful diabetic neuropathy. Overall the Active group had more reduction in pain than the Placebo group. However, this difference varied with the density of the nociceptors in the skin.

### DETAILED DESCRIPTION OF THE INVENTION

### I. Methods of Selection of Patients for Treatment

Alpha-2 adrenergic agonists such as clonidine may be used to treat the pain associated with painful diabetic neuropathy (PDN) and other neuropathies only in a subset of these patients. In one group nociceptors are expressed functionally in the skin and are likely sensitized. This group responds to topical clonidine with significant relief because the targeted alpha-2 adrenergic receptor is expressed in the skin in the nociceptors, activity in which generates the patient's pain. Many patients with PDN have severe degeneration and the targeted nociceptors are not expressed in the skin. The patients still have pain but the pain signaling has moved to proximal levels of the neural axis. If the pain signals are along the nerve, in the dorsal root ganglion, or the central nervous system, then a topical therapy designed to reach the skin is not likely to impact on the patient's pain. It is therefore desirable to have a means to identify the patients that have functional nociceptors in the skin, activity in which is causing at least a portion of the patient's pain. The response to topical clonidine of patients with pain from neuropathy in whom there is severe degeneration does not differ statistically from that seen with placebo. Targeted nociceptors (pain fibers) must be functionally expressed and likely sensitized in the skin in order for clonidine to have a therapeutic effect.

Alpha-2 adrenergic agonists such as clonidine may be used to treat the pain associated with painful diabetic neuropathy (PDN) and other neuropathies only in a subset of these patients. In one group nociceptors are expressed functionally in the skin and are likely sensitized. This group responds to topical clonidine with significant relief because the targeted alpha-2 adrenergic receptor is expressed in the skin in the nociceptors, activity in which generates the patient's pain. Many patients with PDN have severe degeneration and the targeted nociceptors are not expressed in the skin. The presence of the targeted nociceptors may be determined by topical application of a TRPV1 agonist such as capsaicin which induces a sensation of burning pain. Patients who detect the capsaicin as a painful stimulus applied in the area at or near the painful area have expression in the skin of the requisite targeted nociceptors and the targeted alpha-2 adrenergic receptors. The test is referred to as a capsaicin challenge test. The test is based on the understanding that abnormal signaling arises from functional nociceptors in the skin. If there is advanced degeneration in the cutaneous nociceptive afferents, topical capsaicin will evoke little to no pain, as illustrated in Figures 1 and 2. The target for clonidine at the level of the skin in the nociceptors is therefore absent and clonidine does not have a means to affect the abnormal discharge in the "pain" fibers.

Responders can be treated for pain due to length dependent or other neuropathy by local or topical delivery of concentrations of compounds that are agonists of the alpha- 2 adrenergic receptors, especially an alpha₂ adrenergic agonist such as clonidine, to the painful area, without producing systemic levels as appropriate for treating disorders such as hypertension. The compounds of the invention are for use in a method, wherein they are delivered to or adjacent to painful areas in patients who have functional/sensitized nociceptors in the skin. In a patient with painful diabetic neuropathy where the complaint is burning pain in the feet, the alpha-2 agonist of the invention is for use in a method wherein it is topically applied to the feet in the painful region. A preferred formulation for the treatment of patients with painful diabetic neuropathy with expression of functional nociceptors in the targeted region is clonidine applied in an ointment, gel, lotion, spray, or transdermal patch, wherein the dosage is sufficient to provide an effective dose in the painful area or immediately adjacent areas, preferably without producing pharmacologically active systemic blood levels.

The presence of the targeted nociceptors may be determined by topical application of a TRPV1 agonist such as capsaicin, preferably Resiniferatoxin, which induces a sensation of burning pain. Patients who detect the capsaicin as a painful stimulus applied in the area at or near the painful area have expression in the skin of the requisite targeted nociceptors and the targeted alpha-2 adrenergic receptors. The test is referred to as a capsaicin challenge test. The test is based on the understanding that abnormal signaling arises from functional nociceptors in the skin. If there is advanced degeneration in the cutaneous nociceptive afferents, topical capsaicin will evoke little to no pain, as illustrated in Figures 1 and 2. The target for clonidine at the level of the skin in the nociceptors is therefore absent and clonidine does not have a means to affect the abnormal discharge in the "pain" fibers.

This test has been used to determine that there are two subtypes of patients with neuropathic pain. The response to topical clonidine as a therapy depends on the status of this nociceptor innervation as determined with the topical capsaicin challenge test (Figure 2). As can be seen in Figure 2, in one subtype the patients are denervated and do not respond to therapy with topical clonidine. In the other subtype the patients are innervated with nociceptors in the skin. These are referred to as the nociceptor-deafferented group and nociceptor-afferented groups respectively. The two groups have similar amounts of pain and do not differ with respect to a variety of other disease measures.

It has not previously been known that these two subgroups exist, and that there is a way to distinguish the two groups in a clinically useful manner. A method to identify clinical criteria to identify patients who would respond well to this therapy and thus provide clinicians with the means for the rational use of ARC-4558 in PDN patients was developed in the course of developing a topical treatment for PDN, topical clonidine gel (ARC-4558). Topical capsaicin is an ideal way to identify the appropriate patients for treatment. Capsaicin induces a burning pain sensation when applied to the skin in normal subjects. If the subject does not feel capsaicin then the functionality of the nociceptor is in question.

In a Phase IIb, multicenter, randomized, double-blinded, placebo-controlled, parallel-group study of ARC-4558 for the treatment of pain associated with PDN, 179 subjects were randomly assigned in a 1:1 ratio to receive 12 weeks of one of two treatments: clonidine 0.1% gel or placebo gel.

Capsaicin is an example of a TRPV1 agonist that activates nociceptors and induces a burning pain sensation. This is a commonly known property as capsaicin accounts for the burning pain sensation in the mouth when hot peppers are eaten. Capsaicin 0.1 % was applied to the pretibial area between the knee and ankle in each patient. The area was occluded for 30 minutes, at which time patients rated the painfulness of the capsaicin stimulus. It was reasoned that in patients with PDN that the afferented group would detect and rate the capsaicin stimulus as painful, while the deafferented group would fail to detect the stimulus.

Histological techniques can also be used to measure the amount of deafferentation by application of TRPV1 agonist at or near the painful site.

The validity of capsaicin as a test of nociceptor function is evident in the study of the skin biopsy data (Figure 3). Skin biopsies were done near the site where the capsaicin test was applied in 97 of the 179 subjects. The nerve fibers in the epidermis, presumed to be predominantly nociceptors, were quantitatively assessed using the pan-axonal marker PGP 9.5. As shown in Figure 3, the nerve fiber count was significantly lower in the subjects with capsaicin scores of zero versus those with scores above 0 (p<0.05). Thus the biopsy study data show that capsaicin scores correlate with the anatomical demonstration of nociceptors in the skin.

It was discovered that the response to topical capsaicin challenge was a predictive indicator of the reduction in pain resulting from treatment with ARC-4558 (topical clonidine). Figure 4 shows the response to topical clonidine applied to the painful area in patients with different levels of response to the capsaicin challenge test. Subjects who exhibited a positive response to capsaicin challenge (Figures 4B and 4C) were significantly more likely to respond to ARC-4558 than to placebo, while subjects who did not respond to capsaicin challenge were unlikely to have a response that could be differentiated from placebo. Table 1 provides a further breakdown of the analysis by capsaicin score for the change in pain at week 12.

**Table 1. Response to topical clonidine in subjects with painful diabetic neuropathy separated by response to capsaicin challenge text**

| | | |
|---|---|---|
| 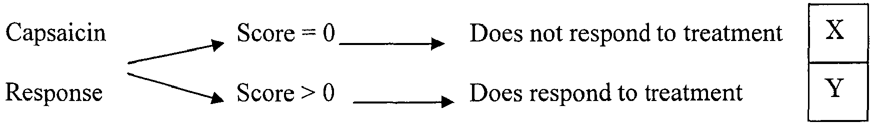 | | |

| Population for Analysis | Mean Difference in Response (Active - Placebo) at Week 12 | P value from the ANCO VA |
|---|---|---|
| ITT | 0.6 | p=0.069 |
| Capsaicin in Score = 0 | 0.5 | p=0.605 |
| Capsaicin in Score > 0 | 0.9 | p=0.046 |
| Capsaicin in Score ≥ 1 | 0.9 | p=0.043 |
| Capsaicin in Score ≥ 2 | 1.2 | p=0.010 |
| Capsaicin in Score ≥ 3 | 1.4 | p=0.001 |
| Capsaicin in Score ≥ 4 | 1.1 | p=0.012 |
| Capsaicin in Score ≥ 5 | 0.9 | p=0.024 |

| | | |
|---|---|---|
| Tables 14-05-01-01-26, 40-47 - BOCF Imputation for Missing Data. ITT = Intention to Treat. | | |

### II. Formulations for Selection of Patients for Treatment

Capsaicin is the pungent ingredient in chili peppers. It is a highly selective agonist for transient receptor potential vanilloid 1 receptor (TRPV1; formerly known as vanilloid receptor 1 (VR1)), a ligand-gated, non-selective cation channel preferentially expressed on small-diameter sensory neurons, especially those C-fibers which specialize in the detection of painful or noxious sensations. TRPV1 responds to noxious stimuli including capsaicin, heat, and extracellular acidification, and will integrate simultaneous exposures to these stimuli. (See: Caterina et al. Annu Rev Neurosci. 2001. 24:487-517). The initial effects of the activation of TRPV1-expressing (capsaicin-sensitive) nociceptors are burning sensations, hyperalgesia, allodynia, and erythema. Analogs of capsaicin with similar physiological properties are known. For example, resiniferatoxin is described as a capsaicin analog by U.S. Patent Nos. 5,290,816, 4,812,446, and 4,424,205. Ton et al., British Journal of Pharmacology, 10, 175-182 (1955) discuss pharmacological actions of capsaicin and its analogs.
In another embodiment, the TRPV1 agonist is specific for TRPA1 receptors. Examples include cinnamaldehyde and allyl isothiocyanate.

The presence, function and/or role of cutaneous generators of the pain in the skin can also be determined by local administration of anesthesia to the skin. In a preferred embodiment, the anesthetic is a local anesthetic such as lidocaine.

The TRV1 agonist or anesthetic can be applied as a solution, ointment, gel, cream, spray or in a device such as a Finn chamber, transdermal patch or wound dressing such as a bandaid.

### III. Formulations for use in Methods for Treatment

The compounds of the invention are for use in a method of treating or reducing the symptoms (i.e. burning, pain) associated with length dependent neuropathies, which includes locally or topically administering an effective amount of an alpha₂-adrenergic agonist or combination thereof. Alpha ₂-adrenergic agonists are known to those skilled in the art. See, for example, The Pharmacological Basis of Therapeutics, 8th Edition, Gill, A. G., T. W. Rall, A. S. Nies, P. Taylor, editors (Pergamon Press, Co., Inc., NY 1990).

Agents with alpha-2 adrenoreceptor agonist activity are represented by Formula I: 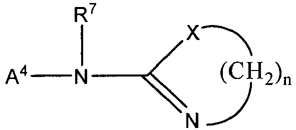 wherein A⁴ may be selected from aryl, and heteroaryl, which may be substituted by one or more radicals selected from alkyl, branched alkyl, cycloalkyl, hydroxyl, alkoxy, cycloalkylalkyl, alkoxyalkyl, aryl, alkanoyl, alkoxycarbonyl, carboxyl, amino, cyano, halogen, thioalkyl, dialkylamino, arylamino, alkylsulfinyl, alkylsulfonyl, arylsulfinyl or arylsulfonyl; wherein X is selected from thio, imino, or methylene; wherein R⁷ is selected from hydrogen, lower alkyl, or oxygen-containing heterocycle; and wherein n is either 2 or 3; or a pharmaceutically acceptable salt thereof.

A preferred class of compounds of Formula I consists of those compounds wherein A⁴ is phenyl; wherein A⁴ is substituted phenyl, on which positions 2 and 6 of the phenyl ring may be independently substituted by a radical selected from hydrogen, chloro, methyl, ethyl, or cycloalkyl, and positions 3, 4, and 5 may be independently substituted by a radical selected from hydrogen, methyl, trifluoromethyl, fluoro, or cyano; wherein A⁴ is 3-thienyl, on which positions 2 and 4 are independently substituted by a radical selected from hydrogen, chloro, methyl, ethyl, or cycloalkyl; wherein A⁴ is 1-naphthyl, 5,6,7,8-tetrahydronaphthyl-1-yl, pyrrolyl, oxazolyl, isoxazolyl, indol-3-yl, indazol-3-yl, quinolinyl, quinazolinyl, quinoxazolinyl, benzoxazolyl, and benzothiophen-3-yl; wherein A⁴ is pyrimidin-4-yl, on which positions 3 and 5 are independently substituted by hydrogen, chloro, methyl, ethyl, cycloalkyl, or methoxy; wherein R⁷ is either hydrogen or tetrahydropyran-2-yl; wherein X is thio or imino; and wherein n is 2.

An especially preferred class of compounds of Formula I consists of compounds wherein A⁴ is selected from phenyl, 2,6-dichlorophenyl, 2,6-dimethylphenyl, 2,6-diethylphenyl, 3,4-dihydroxyphenyl, 3-fluoro-6-methylphenyl, 2-chloro-5-trifluoromethylphenyl, 2-chloro-4-methylphenyl, 3-chloro-4-methylthien-3-yl, 5,6,7,8-tetrahydronaphth-1-yl, and 4-chloro-5-methoxy-2-methylpyrimidin-4-yl; wherein R⁷ is hydrogen or tetrahydropyran-2-yl; wherein X is thio or imino; and wherein n is 2. A specifically preferred class of compounds of Formula I consists of xylazine, flutonidine, moxonidine, tramazoline, tolonidine, piclonidine, tiamenidine, and clonidine.

Topical administration is described for treatment of sympathetically maintained pain in U.S. Patent No. 5,447,947 issued September 5, 1995 to Campbell, and in U.S. Patent Nos. 6,534,048 issued March 18, 2003 to Borgman and 6,147,102 issued November 15, 2000 to Borgman.
In the method described herein, the compounds are administered locally or topically directly to or adjacent the painful area, in a suitable pharmaceutical carrier, many of which are known to those skilled in the art. The carrier can be in the form of a lotion, ointment, gel, solution, or transdermal patch, or a topical spray. The topical application allows the drug to reach high concentration at the painful area or tissue immediately adjacent thereto, avoiding many of the side effects of these compounds observed following systemic administration.

Formulation of drugs is discussed in, for example, Hoover, John E., Remington's Pharmaceutical Sciences, Mack Publishing Co., Easton, Pennsylvania (1975), and Liberman, H.A. and Lachman, L., Eds., Pharmaceutical Dosage Forms, Marcel Decker, New York, N.Y. (1980). Pharmaceutical compositions may be formulated in conventional manner using one or more physiologically acceptable carriers comprising excipients and auxiliaries which facilitate processing of the active compounds into preparations which can be used pharmaceutically.

The preferred embodiment of the formulation consists of:
Clonidine hydrochloride USP 0.1%
Benzyl alcohol NF 1.0%
Carbopol 980 NF 0.6%
Sodium hydroxide NF adjust to pH 8
Hydrochloric acid NF adjust to pH 8 (if necessary)
Purified water USP qs ad 100%

Patients may be screened with the capsaicin test. Patients who report pain to the capsaicin test will have an excellent chance of responding favorably to the topical alpha-2 adrenergic agonist treatment.

The compounds of the invention are for use in a method of treating or reducing the symptoms (i.e. burning, pain) associated with neuropathies, which includes locally or topically administering an effective amount of an alpha₂-adrenergic agonist to the painful site. This screening clarifies the group of patient that will respond to this therapy. The therapy is not effective in all patients with PDN, but rather only works when it is applied to the patients that have innervations.

The dosage formulation of the invention is for use in a method, wherein it is administered from once a day to several times a day, depending on the patient. In one embodiment, the therapeutic agent of the invention is clonidine for use in a method wherein it is administered in a concentration between 0.05 and 10% clonidine. The dose is determined by the region of pain. Because the effect of the clonidine is local it must be applied to the painful area. Thus in patients with broader areas of pain a higher dose of clonidine will be necessary though the percent concentration remains constant. The area treated is constrained by the systemic dosing. In the study done with 0.1 % and 0.2% clonidine, the mean blood level was well below 0.1 ng/mg (one third of patients had no detectable clonidine in the blood), whereas the blood levels exceed 0.2 ng/ml with systemic delivery.

In study CLO-027, blood samples for PK analysis were obtained at baseline, and at Weeks 2 and 12 of treatment. The blood levels of clonidine were below the limit of detection in more than 75% of the subjects at both weeks 2 and weeks 12 (limit of detection for the clonidine assay was 0.010 ng/mL). The mean blood level at two weeks was 0.017 ng/mL (n=83; SD 0.024). Excluding one outlier, the mean level at week 12 was 0.019 ng/mL (n=79; SD 0.038). Thus the 2 and 12 week PK levels were nearly identical and several standard deviations below the lower threshold value considered necessary to treat hypertension (0.200 ng/ml).

Figures 4A-4C are graphs of the data from 179 subjects with painful diabetic neuropathy with pain in the feet enrolled in a 12 week double blinded randomized clinical trial to assess the analgesic efficacy of topical clonidine applied to the painful area. Pain was assessed each day using a pain diary using a standardized numerical pain rating scale (NPRS). The average pain for each week was calculated and the difference from baseline was determined (week x minus baseline). Figures 4A, B, and C show the change in pain over time for placebo and active (clonidine treatment group) for different subgroups based on ratings of the 30 minute capsaicin challenge test. Shown in Figure 4A are the results in subjects who felt no pain to the capsaicin stimulus. Figures 4B and 4C show the results for subjects with capsaicin responses greater than 0 and ≥2, respectively. The baseline pain scores were imputed for missing data arising from premature withdrawal from the study (BOCF, baseline observation carried forward). The numbers of subjects for each group are shown in the upper right-hand corner. These data demonstrated that clonidine had no efficacy over placebo in patients who did not detect the capsaicin stimulus. However, in subjects who detected capsaicin (pain rating>0), or even more clearly, had a capsaicin pain rating of two or more (0 to 10 scale) clonidine was significantly superior to placebo in relieving the diabetic pain.

Figure 5 indicates the reduction in pain (week 12 of treatment compared to baseline) in the Clonidine Gel group versus the Placebo group as a function of nerve fiber density (nociceptors) in the superficial layer of the skin (epidermis). The data were collected in a 97 patient subgroup as part of a larger double blind randomized study in 180 patients with painful diabetic neuropathy. Overall the Active group had more reduction in pain than the Placebo group. However, this difference varied with the density of the nociceptors in the skin. The topical clonidine had greater efficacy over placebo in patients with greater concentration of pain fibers in the skin. This further supports the concept that clonidine applied topically has efficacy that relates to the presence of the targeted nociceptors in the skin.

Unless defined otherwise, all technical and scientific terms used herein have the same meanings as commonly understood by one of skill in the art to which the disclosed invention belongs. Although any methods and materials similar or equivalent to those described herein can be used in the practice or testing of the present invention, the preferred methods devices, and materials are as described. Those skilled in the art will recognize or be able to ascertain using no more than routine experimentation many equivalents to the specific embodiments of the invention described herein. Such equivalents are intended to be encompassed by the following claims.

## Claims

1. An alpha-2 adrenergic agonist for use in a method of treating pain in a patient suffering from painful diabetic neuropathy wherein
first the presence of nociceptor function is determined in the patient in a manner that identifies those patients, which would show an evoked pain level of >0 on a 0-10 numerical pain rating scale of the capsaicin challenge test when being subjected to a 0.1% capsaicin stimulus applied to the pretibial area for 30 minutes; and then,
if the patient has nociceptor function in the skin, said alpha-2 adrenergic agonist is topically applied at and/or in the area described as painful.

2. The alpha-2 adrenergic agonist for use of claim 1 comprising using histological techniques to measure the amount of deafferentation by application of TRPV1 agonist at or near the painful site.

3. The alpha-2 adrenergic agonist for use of claim 1 wherein the presence, function and/or role of cutaneous generators of the pain in the skin is determined by local administration of anesthesia to the skin.

4. The alpha-2 adrenergic agonist for use of claim 3 wherein the anesthetic is a local anesthetic.

5. The alpha-2 adrenergic agonist for use of claim 1, wherein the individuals have a capsaicin challenge test score greater than or equal to 2.

6. The alpha-2 adrenergic agonist for use of claim 1, wherein the individuals have a capsaicin challenge test score greater than or equal to 3.

7. The alpha-2 adrenergic agonist for use of claim 1, wherein the individuals have a capsaicin challenge test score greater than or equal to 4.

8. The alpha-2 adrenergic agonist for use of claim 1, wherein the individuals have a capsaicin challenge test score greater than or equal to 5.

## Patentansprüche

1. Alpha-2-adrenerger Agonist zur Verwendung bei einem Verfahren zur Behandlung von Schmerz bei einem Patienten, der an schmerzhafter diabetischer Neuropathie leidet, wobei
zuerst das Vorlieben einer Nociceptor-Funktion bei dem Patienten in einer Weise bestimmt wird, die solche Patienten identifiziert, welche ein evoziertes Schmerzniveau von > 0 auf einer numerischen Schmerzbewertungsskala von 0 bis 10 des Capsaicin-Challenge-Tests zeigen würden, wenn sie einem 0,1%-Capsaicin-Stimulus, appliziert auf den prätibialen Bereich über 30 Minuten, ausgesetzt werden; und dann,
wenn der Patient eine Nociceptor-Funktion in der Haut aufweist, der genannte Alpha-2-adrenerge Agonist topisch auf und/oder in dem als schmerzhaft beschriebenen Bereich appliziert wird.

2. Alpha-2-adrenerger Agonist zur Verwendung gemäß Anspruch 1, umfassend Verwendung histologischer Techniken zum Messen des Deafferentierungsgrades durch Applikation eines TRPV1-Agonisten auf oder in der Nähe der schmerzhaften Stelle.

3. Alpha-2-adrenerger Agonist zur Verwendung gemäß Anspruch 1, wobei das Vorliegen, die Funktion und/oder die Rolle kutaner Generatoren des Schmerzes in der Haut durch lokale Verabreichung einer Anästhesie an die Haut bestimmt werden.

4. Alpha-2-adrenerger Agonist zur Verwendung gemäß Anspruch 3, wobei das Anästhetikum ein Lokalanästhetikum ist.

5. Alpha-2-adrenerger Agonist zur Verwendung gemäß Anspruch 1, wobei die Individuen einen Capsaicin-Challenge-Testwert größer oder gleich 2 aufweisen.

6. Alpha-2-adrenerger Agonist zur Verwendung gemäß Anspruch 1, wobei die Individuen einen Capsaicin-Challenge-Testwert größer oder gleich 3 aufweisen.

7. Alpha-2-adrenerger Agonist zur Verwendung gemäß Anspruch 1, wobei die Individuen einen Capsaicin-Challenge-Testwert größer oder gleich 4 aufweisen.

8. Alpha-2-adrenerger Agonist zur Verwendung gemäß Anspruch 1, wobei die Individuen einen Capsaicin-Challenge-Testwert größer oder gleich 5 aufweisen.

## Revendications

1. Agoniste alpha-2-adrénergique destiné à être utilisé dans une méthode de traitement de la douleur chez un patient souffrant d'une neuropathie diabétique douloureuse, dans lequel
la présence d'une fonction de nocicepteur chez un patient est tout d'abord déterminée de manière à identifier ces patients qui montreraient un niveau de douleur suscité > 0 sur une échelle d'évaluation numérique de la douleur allant de 0-10 d'un essai de stimulation à la capsaïcine, lorsqu'ils sont soumis à un stimulus à de la capsaïcine à 0,1% appliquée sur la région prétibiale pendant 30 minutes ; et ensuite,
si le patient possède une fonction de nocicepteur dans la peau, ledit agoniste alpha-2-adrénergique est appliqué d'une manière topique sur et/ou dans la région définie comme étant douloureuse.

2. Agoniste alpha-2-adrénergique destiné à être utilisé selon la revendication 1, comprenant l'utilisation de techniques histologiques pour mesurer la quantité de désafférentation par application de l'agoniste TRPV1 sur ou à proximité du site de la douleur.

3. Agoniste alpha-2-adrénergique destiné à être utilisé selon la revendication 1, dans lequel la présence, la fonction et/ou le rôle de générateurs cutanés de la douleur dans la peau est ou sont déterminé(e)(s) par l'administration locale d'anesthésique sur la peau.

4. Agoniste alpha-2-adrénergique destiné à être utilisé selon la revendication 3, dans lequel l'anesthésique est un anesthésique local.

5. Agoniste alpha-2-adrénergique destiné à être utilisé selon la revendication 1, dans lequel les individus ont un score à l'essai de stimulation à la capsaïcine supérieur ou égal à 2.

6. Agoniste alpha-2-adrénergique destiné à être utilisé selon la revendication 1, dans lequel les individus ont un score à l'essai de stimulation à la capsaïcine supérieur ou égal à 3.

7. Agoniste alpha-2-adrénergique destiné à être utilisé selon la revendication 1, dans lequel les individus ont un score à l'essai de stimulation à la capsaïcine supérieur ou égal à 4.

8. Agoniste Ipha-2-adrénergique destiné à être utilisé selon la revendication 1, dans lequel les individus ont un score à l'essai de stimulation à la capsaïcine supérieur ou égal à 5.
